Europäisches Patentamt

European Patent Office

Office européen des brevets

(19)

(11) Publication number: **0 351 173**
**A2**

# EUROPEAN PATENT APPLICATION

(21) Application number: **89307000.3**

(22) Date of filing: **10.07.89**

(51) Int. Cl.⁴: **C07H 17/08 , A61K 31/70**

Claims for the following Contracting States: ES + GR.

(30) Priority: **14.07.88 JP 176133/88**

(43) Date of publication of application:
**17.01.90 Bulletin 90/03**

(84) Designated Contracting States:
**AT BE CH DE ES FR GB GR IT LI LU NL SE**

(71) Applicant: **OSAKA PREFECTURE**
**1-22 Otemae 2-chome Chuo-ku**
**Osaka-shi(JP)**

Applicant: **TANABE SEIYAKU CO., LTD.**
**2-10, Dosho-machi 3-chome**
**Chuo-ku Osaka(JP)**

(72) Inventor: **Otake, Toru**
**22-26, Sekiyakita 7-chome Kashiba-cho**
**Kitakatsuragi-gun Nara-ken(JP)**
Inventor: **Mori, Haruyo**
**2-12, Akanedai 1-chome Haibara-cho**
**Uda-gun Nara-ken(JP)**
Inventor: **Ueba, Noboru**
**81-10, Izumihara-cho**
**Yamatokoriyama-shi Nara-ken(JP)**
Inventor: **Miyano, Keiichi**
**19-6, Kuranouchi**
**Habikino-shi Osaka-fu(JP)**
Inventor: **Matsumoto, Kazuo**
**23-27, Yamate-dai 5-chome**
**Ibaraki-shi Osaka-fu(JP)**
Inventor: **Moriya, Tamon**
**5-9, Kitashowadai-cho**
**Takatsuki-shi Osaka-fu(JP)**

(74) Representative: **Hardisty, David Robert et al**
**BOULT, WADE & TENNANT 27 Furnival Street**
**London EC4A IPQ(GB)**

(54) Substance having anti-retrovirus activity.

(57) A reaction product of Amphotericin B with a sulfonating agent, having an excellent anti-retrovirus activity, especially an excellent HIV proliferation inhibitory activity. The substance of the present invention can be used for prevention and/or treatment of a retrovirus infection diseases such as AIDS.

EP 0 351 173 A2

## Substance Having Anti-Retrovirus Activity

The present invention relates to a substance having an anti-retrovirus activity, more particularly, to a reaction product of Amphotericin B with a sulfonating agent, or a salt thereof, which has a retrovirus proliferation-inhibitory activity.

### Technical Background and Prior Art

AIDS (acquired immune deficiency syndrome) is a lethal or extremely malignant disease caused by infection of human immunodeficiency virus (HIV), a kind of retrovirus. To prevent and eradicate this disease is now the most important task to be achieved for human being in all of the world.

Hitherto, there have been attempts to treat AIDS with an anti-retrovirus agent which inhibits an activity of reverse transcriptase of HIV. Typical example of such anti-retrovirus agent includes, for example, 3'-azidothymidine (AZT) [cf. "Igaku no Ayumi" (Progress in Medicine), 142, No. 9, 619-622 (1987)].

However, the conventional anti-retrovirus agents have not yet been proved or confirmed for their effectiveness and safety in the treatment of AIDS.

### Brief Summary of the Invention

Under such circumstances, the present inventors have intensively studied to develop an anti-retrovirus agent which is effective and safe in the treatment of AIDS. As a result, it has been found that a reaction product of Amphotericin B with a sulfonating agent, or a salt thereof, has an excellent anti-retrovirus activity, especially an HIV proliferation-inhibitory activity.

### Brief Description of the Drawing

Fig 1 and Fig. 2 are IR absorption spectra and high performance liquid chromatogram profiles of the substance obtained in Example 1, respectively.

Fig. 3 and Fig. 4 are IR absorption spectra and high performance liquid chromatogram profiles of the substance obtained in Example 2.

### Detailed Description of the Invention

The reaction product of Amphotericin B with a sulfonating agent of the present invention can be prepared, for example, by reacting Amphotericin B and the sulfonating agent in a suitable solvent. Preferable solvent includes, for example, a tertiary amine (e.g. pyridine, picoline, lutidine, N,N-dimethylaniline, etc.). The sulfonating agent used in the present invention includes, for example, sulfur trioxide-pyridine complex, sulfuric anhydride, conc. sulfuric acid, chlorosulfuric acid, and the like. The sulfonating agent is preferably employed in an excess amount to Amphotericin B. For example, when the sulfonating agent is sulfur trioxide-pyridine complex, 5 to 100 moles of the sulfonating agent are preferably used per 1 mole of Amphotericin B. The reaction can be carried out under cooling, at room temperature or under heating, preferably under heating.

For example, using pyridine as the solvent and sulfur trioxide-pyridine complex as the sulfonating agent, the reaction of Amphotericin B with the sulfonating agent affords an oily product. Since this oily product contains impurities including pyridine used as the solvent, the unreacted sulfur trioxide-pyridine complex of the sulfonating agent and the like, it is preferable to remove such impurities. The removal of the impurities can be conducted, for example, by dissolving the above oily product in water, treating the solution with a strong acid ion exchange resin to remove pyridine of basic materials, adding barium hydroxide and/or barium carbonate to the solution to precipitate sulfate ion as barium sulfate, and then removing the precipitate. Further, the desired product can be obtained as an alkali metal salt by treating the solution obtained after removal of barium sulfate with a strong acid ion exchange resin to remove barium ion, adding an alkali metal hydroxide (e.g. potassium hydroxide) to the solution, and removing the insoluble materials, followed by lyophilization.

The thus obtained substance of the present invention has the following physicochemical properties:

2

(1) IR absorption spectra by KBr tablet method:

Absorption band at around 3450-3420, 1630, 1240, 1120-1110 and 620 cm⁻¹

    (2) Ratio of carbon, nitrogen and sulfur atoms:

C : N : S = 21-22 : 1 : 3-4

    (3) Solubility:

Highly soluble in water, hardly soluble in ethanol, and insoluble in ethyl acetate and ether.

Among the above substances of the present invention, a potassium salt is most preferable. The potassium salt obtained in Examples described hereinafter has the following physicochemical properties:

Substance obtained in Example 1:

    (1) IR absorption spectra by KBr tablet method:

Absorption band at around 3450, 1630, 1240, 1120 and 620 cm⁻¹

    (2) High performance liquid chromatogram:

[conditions; stationary phase: octadecanylated silica-gel, 15 cm x 4.6 mm$\phi$, mobile phase: methanol : water = 40 : 60, column temperature: 25°C, detector: UV 254 nm, flow rate: 0.5 ml/min.]

Rt (retention time(min.) of main peak: around 1.9 Substance obtained in Example 2:

    (1) IR absorption spectra by KBr tablet method:

Absorption band at around 3420, 1630, 1240, 1110 and 620 cm⁻¹

    (2) High performance liquid chromatogram:

[conditions; stationary phase: octadecanylated silica-gel, 15 cm x 4.6 mm$\phi$, mobile phase: methanol : water = 40 : 60, column temperature: 25°C, detector: UV 254 nm, flow rate: 0.5 ml/min.]

Rt (retention time)(min.) of main peak: around 1.8

The reaction product of Amphotericin B with the sulfonating agent, or the salt thereof, of the present invention has an excellent anti-retrovirus activity, especially an excellent HIV proliferation-inhibitory activity, as mentioned above, and thus, can be used for prevention and/or treatment of retrovirus infectious diseases such as AIDS.

The reaction product of Amphotericin B with the sulfonating agent, or the salt thereof, of the present invention can be administered either orally or parenterally (e.g. intravenously, subcutaneously, etc.). The daily dose of the reaction product or the salt thereof of the present invention may vary over a wide range depending on the age, weight or conditions of patients, and the severity of diseases to be treated. In general, a preferred daily dose of said reaction product or the salt thereof may be about 0.1 to about 300 mg, especially 0.5 to 100 mg, per kg of body weight per day. Further, the reaction product and the salt thereof of the present invention may be used in the form of a pharmaceutical preparation containing the substance in conjunction or admixture with pharmaceutical excipients suitable for oral or parenteral administration. Suitable excipients include, for example, gelatin, lactose, glucose, sodium chloride, starch, magnesium stearate, talcum, vegetable oil and other known medicinal excipients. The pharmaceutical preparations may be in solid form such as tablets, coated tablets, pills or capsules; or in liquid form such as solutions, suspensions or emulsions. They may be sterillized and/or may further contain auxiliaries such as stabilizing, wetting or emulsifying agents.

The present invention is illustrated by the following Experiments and Examples, but should not be construed as limited thereto.

Experiment 1 (HIV proliferation-inhibitory activity)

It is known that when MT-4 cell, which is a persistently infected cell line of human T-cell Leukemia virus (HTLV) type I (hereinafter referred to as "ATLV"), is infected with HIV, HIV rapidly proliferates and the MT-4 cell dies within 5 to 6 days due to cytotoxicity. Therefore, a viable cell number of the HIV-infected MT-4 cell can be employed as a measure for the evaluation of HIV proliferation-inhibitory activity of specimens.

MT-4 cells were infected with HIV (culture supernatant of TALL-1/LAV) at 37°C for 1 hour so that 0.001 of TCID$_{50}$ (median tissue culture infectious dose) per cell is obtained, washed and resuspended in RPMI-1640 medium containing 20 % FCS (fetal calf serum) at a concentration of 2 x 10⁵ cells/ml. Specimens diluted with RPMI-1640 medium containing no FCS (in serial fold dilutions) were put on flat-bottomed culture plate with 8 wells per one concentration in an amount of 100 μl/well. To half (4 wells) of these 8 wells were added each 100 μl/well of suspensions of the above MT-4 cells infected with HIV, and to the remaining 4 wells were added each 100 μl/well of suspensions of MT-4 cells not infected with HIV, followed by culture for 5 days. After culture, 100 μl of cell suspensions were taken from each well and subjected to counting of a viable cell number by a trypan blue dyeing method. The HIV proliferation-inhibitory activity of specimens was evaluated on the basis of percentage of citotoxicity inhibition calculated by the following equation:

Percentage of Citotoxicity Inhibition (%)

$$= \frac{\text{Viable Cell Number of HIV-infected MT-4 cell}}{\text{Viable Cell Number of HIV-non-infected MT-4 cell}} \times 100$$

The results are shown in Table 1 which demonstrates that the substance of the present invention exhibits more than 70 % of citotoxicity inhibition even at such a low concentration of 7.5 $\mu$g/ml, and thus, has an excellent HIV proliferation-inhibitory activity.

Table 1

| | Percentage of citotoxicity inhibition (%) | | | | |
|---|---|---|---|---|---|
| | Conc. of specimens ($\mu$g/ml) | | | | |
| | 0 | 1.88 | 3.75 | 7.5 | 15 |
| Spec. 1 | 4 | 9 | 21 | 73 | 100 |
| Spec. 2 | 4 . | 16 | 36 | 88 | (100)* |
| (Note) | | | | | |
| Spec. 1: Substance obtained in Example 1 | | | | | |
| Spec. 2: Substance obtained in Example 2 | | | | | |

*: It is described as (100) when the data exceed 100 due to dispersion of measurement.

Experiment 2 (Giant Cell Formation Inhibitory Activity)

When Molt-4 cell is co-cultured with Molt-4/HIV cell which is persistant-infected with HIV, a giant cell is formed within 1 to 2 days. This phenomenon is caused by linkage between CD4 receptor on the surface of Molt-4 cell and HIV envelope protein gp120 produced on the surface of Molt-4/HIV cell. Therefore, inhibitory activity on linkage between specimen HIV and CD4 molecule (adsorption of HIV to lymphocyte) can be evaluated by the presence of giant cell formation.

To each well of flat-bottomed culture plate were added a serially diluted solution of specimen (100 $\mu$l) and a cell suspension of a mixture of Molt-4 cell and Molt-4/HTLV-III cell (9:1)(6 x $10^5$ cells/ml, 100 $\mu$l), followed by culture. After 2 days, the presence of giant cells was observed with microscope. As a result, it was found that the substances obtained in Examples 1 and 2 could inhibit the giant cell formation by 100 % at a concentration of 31 $\mu$g/ml.

Experiment 3 (Reverse Transcriptase Inhibitory Activity)

This experiment was carried out in accordance with a method of Lee et al. [J. Clinc. Microbio. 25, 1717, (1987)]. That is, a culture supernatant (100 ml) of Molt-4/LAV cell, which is a persistent infection cell of HIV, was centrifuged, and to the precipitate was added virus-solubilizing buffer (10 ml) to prepare a solution having a reverse transcriptase activity. To 50 $\mu$l of this solution was added a serially diluted solution (50 $\mu$l) of specimen, and the mixture was reacted for 30 minutes, and thereto was added a buffer solution for reverse transcriptase (RT) reaction (90 $\mu$l). 95 $\mu$l thereof was added to a tube containing $^3$H-thymidine triphosphate (10 $\mu$Ci) and a template primer [poly(rA)$\cdot$p(dT)$_{12-18}$](2.5 $\mu$g/5 $\mu$l)(total amount: 100 $\mu$l). The tube was covered and left to stand for reaction at 37°C for 22 hours. After the reaction was stopped with addition of a 10 % aqueous solution of trichloroacetic acid, the reaction mixture was filtered through a glass filter and the unreacted $^3$H-thymidine triphophate was removed by washing with a 5 % aqueous solution of trichloroacetic acid. The glass filter was dried and subjected to measurement of radioactivity with a liquid scintilation counter to evaluate a reverse transcriptase activity. The evaluation of the reverse transcriptase was made based on percentage (%) of reverse transcriptase inhibition calculated by the following equation:

$$\text{Percentage of RT Inhibition (\%)} = \frac{P_1 - P_2}{P_1} \times 100$$

$P_1$: Enzyme activity when specimen was not added.

$P_2$: Enzyme activity when specimen was added.

The results are shown in Table 2 which demonstrates that the substance of the present invention has an excellent reverse transcriptase inhibitory activity.

Table 2

| | Percent of Rt Inhibition (%) | | | |
|---|---|---|---|---|
| | Conc. of specimen (μg/l) | | | |
| | 40 | 200 | 1,000 | 5,000 |
| Spec. 1 | -0.7 | 49.9 | 96.5 | 99.8 |
| Spec. 2 | 28.5 | 78.7 | 98.7 | 99.9 |
| (Note) | | | | |
| Spec. 1: Substance obtained in Example 1. | | | | |
| Spec. 2: Substance obtained in Example 2. | | | | |

Example 1

Amphotericin B (0.50 g) is dissolved in pyridine (100 ml) and to the solution are added sulfur trioxide-pyridine complex (10 g) and then pyridine (100 ml). The mixture is stirred at 60 to 65°C for 6 hours. After cooling, the supernate is removed and the residue is dissolved in water (15 ml). To the solution is added a strong acid ion exchange resin [Dowex 50X8 (H type)](100 ml, in wet) and the mixture is stirred for 30 minutes. The resin is filtered off and the filtrate is adjusted to pH 5.5 with a 10 % aqueous barium hydroxide solution and then to pH 7.5 with powdery barium carbonate. After addition of active carbon, the insoluble material is filtered off with Celite. To the filtrate is added a strong acid ion exchange resin [Dowex 50X8 (H type)](30 ml, in wet) and the mixture is stirred for 20 minutes. After removal of the resin by filtration, the filtrate is adjusted to pH 7.3 with a 0.2 % aqueous solution of potassium hydroxide, and the solution is concentrated to about 100 ml of volume. After treatment with active carbon, the solution is lyophilized to give 185 mg of a substance having the following physicochemical properties (a reaction product of Amphotericin B with the sulfonating agent, potassium salt):

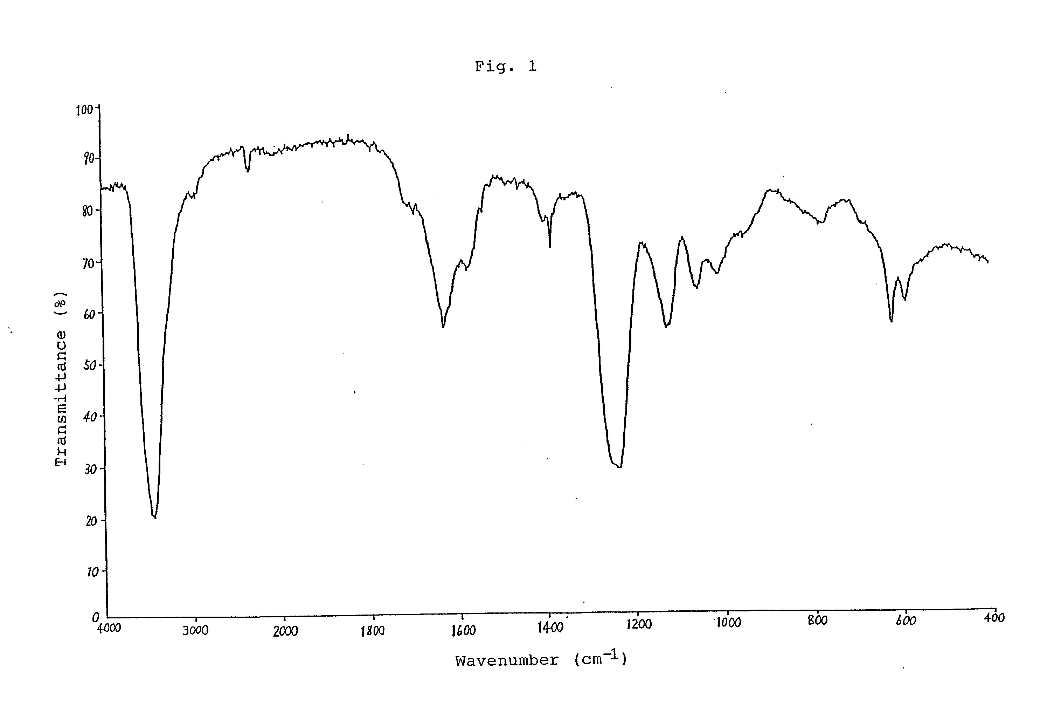

(1) IR absorption spectra (in KBr)(Fig. 1): 3450, 1630, 1240, 1120, 620 cm$^{-1}$.

(2) UV absorption spectrum (methanol): the spectrum inherent in Amphotericin B disappears.

(3) Elementary analysis:

Found (%): C, 24.97; H, 2.82; N, 1.39; S, 11.51; K, 20.64

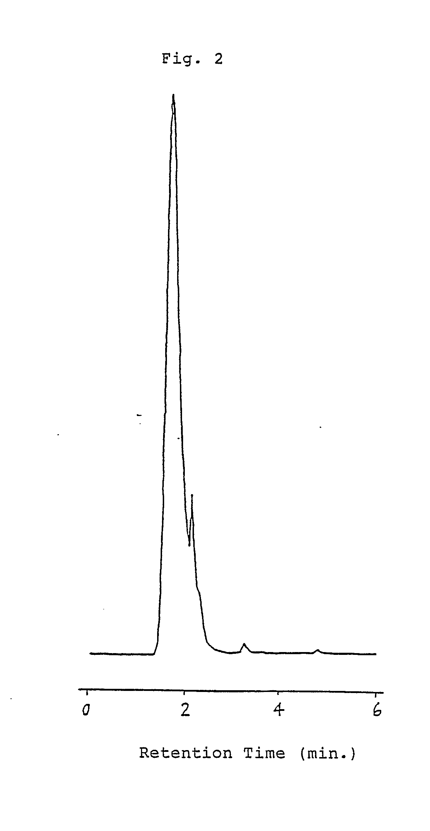

(4) High performance liquid chromatogram (Fig. 2):

Rt (retention time)(min.) of main peak: around 1.9.

[Conditions]

Stationary phase: octadecanylated silica-gel (15 cm x 4.6 mmφ)

Mobile phase: methanol : water = 40 : 60

Column temperature: 25°C

Detector: UV 254 nm

Flow rate: 0.5 ml/min.
    (5) Solubility:
Highly soluble in water, hardly soluble in ethanol, and insoluble in ethyl acetate and ether.


Example 2

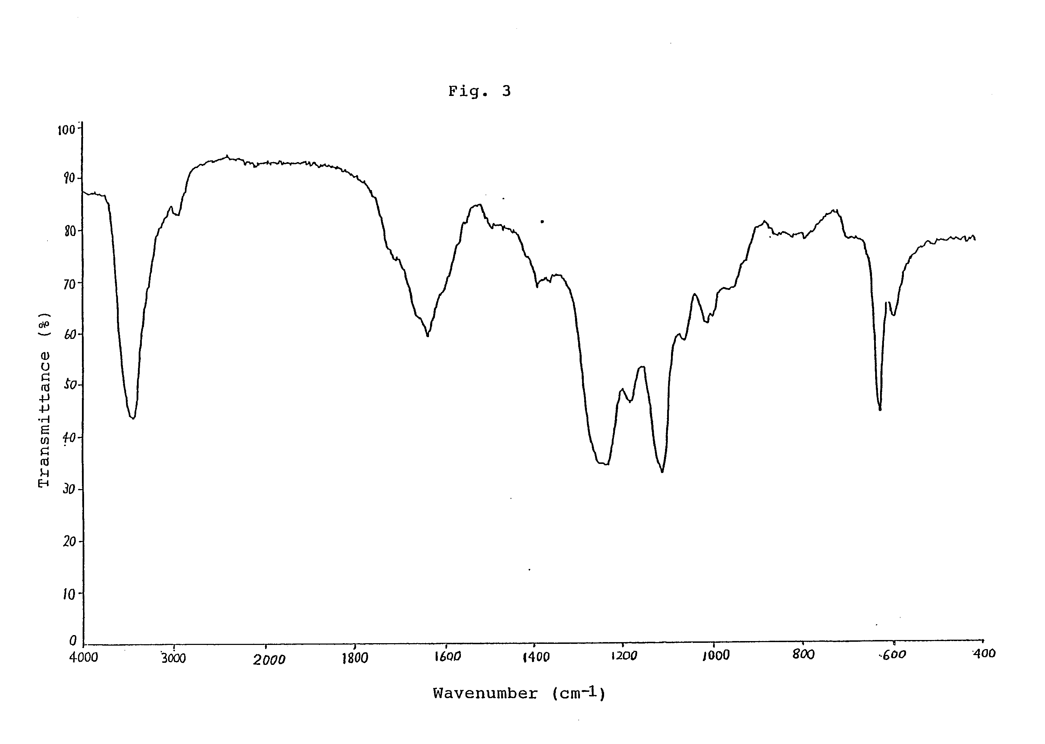

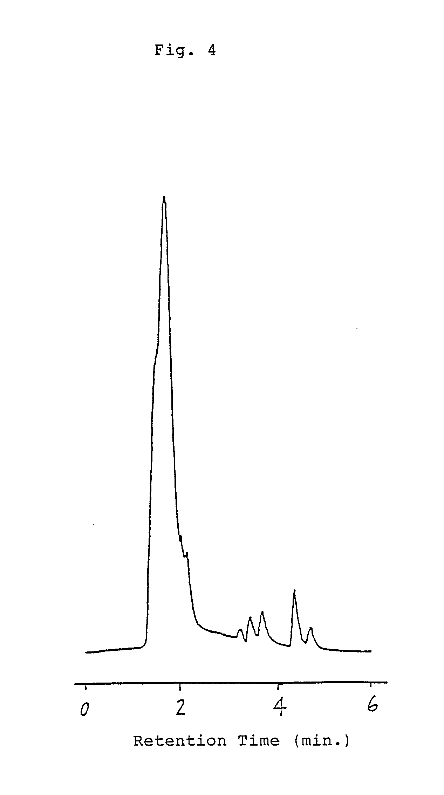

    Amphotericin B (0.50 g) is dissolved in pyridine (100 ml) and to the solution are added sulfur trioxide-pyridine complex (10 g) and then pyridine (100 ml). The mixture is stirred at 70°C for 6 hours. After cooling, the supernate is removed and the residue is dissolved in water 15 ml). To the solution is added a strong acid ion exchange resin [Dowex 50X8 (H type)](100 ml, in wet) and the mixture is stirred for 20 minutes. The resin is filtered off and the filtrate is adjusted to pH 5.5 with an aqueous solution of barium hydroxide and then to pH 7.5 with powdery barium carbonate. The precipitates are centrifuged (7,000 rpm, 20 min.). To the supernatant is added active carbon and the mixture is filtered. To the filtrate is added a strong acid ion exchange resin [Dowex 50X8 (H type)](15 ml, in wet) and the mixture is stirred for 15 minutes, followed by filtration of insoluble materials. After the filtrate is adjusted to pH 7.3 with a 1 % aqueous solution of potassium hydroxide, the solution is concentrated to about 50 ml of volume. After filtration with a membrane filter, the filtrate is lyophilized to give 250 mg of a substance having the following physicochemical properties (a reaction product of Amphotericin B with the sulfonating agent, potassium salt):
    (1) IR absorption spectra (in KBr)(Fig. 3): 3420, 1630, 1240, 1110, 620 cm$^{-1}$.
    (2) UV absorption spectrum (methanol): the spectrum inherent in Amphotericin B disappears.
    (3) Elementary analysis:
Found (%): C, 24.72; H, 2.96; N, 1.34; S, 11.83; K, 20.02
    (4) High performance liquid chromatogram (Fig. 4):
Rt (retention time)(min.) of main peak: around 1.8
[Conditions]
Stationary phase: octadecanylated silica-gel (15 cm x 4.6 mm$\phi$)
Mobile phase: methanol : water = 40 : 60
Column temperature: 25°C
Detector: UV 254 nm
Flow rate: 0.5 ml/min.
    (5) Solubility
Highly soluble in water, hardly soluble in ethanol, and insoluble in ethyl acetate and ether.


**Claims**

    1. A reaction product of Amphotericin B with a sulfonating agent, or a pharmaceutically acceptable salt thereof.
    2. The reaction product of claim 1 which has an anti-retrovirus activity.
    3. The reaction product of claim 1 which has an HIV proliferation-inhibitory activity.
    4. The reaction product of claim 1 which has the following physicochemical properties:
        (1) IR absorption spectra by KBr tablet method:
Absorption band at around 3450-3420, 1630, 1240, 1120-1110 and 620 cm$^{-1}$
        (2) Ratio of carbon, nitrogen and sulfur atoms:
C : N : S = 21-22 : 1 : 3-4
        (3) Solubility:
Highly soluble in water, hardly soluble in ethanol, and insoluble in ethyl acetate and ether.
    5. The reaction product of claim 1 which has the following physicochemical properties:
        (1) IR absorption spectra by KBr tablet method:
Absorption band at around 3450, 1630, 1240, 1120 and 620 cm$^{-1}$.
        (2) High performance liquid chromatogram:
[conditions; stationary phase: octadecanylated silica-gel, 15 cm x 4.6 mm$\phi$, mobile phase: methanol : water = 40 : 60, column temperature: 25°C, detector: UV 254 nm, flow rate: 0.5 ml/min.]
Retention time of main peak: around 1.9 minutes.
    6. The reaction product of claim 1 which has the following physicochemical properties:
        (1) IR absorption spectra by KBr tablet method:

Absorption band at around 3420, 1630, 1240, 1110 and 620 cm$^{-1}$.

(2) High performance liquid chromatogram:

[conditions; stationary phase: octadecanylated silica-gel, 15 cm x 4.6 mm$\phi$, mobile phase: methanol : water = 40 : 60, column temperature: 25°C, detector: UV 254 nm, flow rate: 0.5 ml/min.]

Retention time of main peak: around 1.8 minutes.

7. A pharmaceutical composition which comprises a therapeutically effective amount of a reaction product of Amphotericin B with a sulfonating agent, or a pharmaceutically acceptable salt thereof and a pharmaceutically acceptable carrier therefor.

8. An anti-retrovirus agent comprising a reaction product of Amphotericin B with a sulfonating agent, or a pharmaceutically acceptable salt thereof as an active therapeutic substance.

9. The use of the agent of claim 8 for the manufacture of a medicament for the therapeutic treatment of the acquired immune deficiency syndrome (AIDS).

10. The use of the agent of claim 8 for the manufacture of a medicament for the therapeutic treatment of the human immunodeficiency virus (HIV) infection.

Claims for the following Contracting States: GR, ES

1. A method of producing a therapeutic agent which comprises reacting Amphotericin B with a sulfonating agent, or a pharmaceutically acceptable salt thereof to produce a reaction product.

2. A method as claimed in claim 1 wherein the reaction product has an anti-retrovirus activity.

3. A method as claimed in claim 1 wherein the reaction product has an HIV proliferation-inhibitory activity.

4. A method as claimed in claim 1 wherein the reaction product has the following physicochemical properties:

(1) IR absorption spectra by KBr tablet method:

Absorption band at around 3450-3420, 1630, 1240, 1120 1110 and 620 cm 1.

(2) Ratio of carbon, nitrogen and sulfur atoms:

C : N : S = 21-22 : 1 : 3-4.

(3) Solubility:

Highly soluble in water, hardly soluble in ethanol, and insoluble in ethyl acetate and ether.

5. A method as claimed in claim 1 wherein the reaction product has the following physicochemical properties:

(1) IR absorption spectra by KBr tablet method:

Absorption band at around 3450, 1630, 1240, 1120 and 620 cm$^{-1}$.

(2) High performance liquid chromatogram:

[conditions; stationary phase:

octadecanylated silica-gel, 15 cm x 4.6 mm$\phi$, mobile phase: methanol : water = 40 : 60, column temperature: 25°C, detector: UV 254 nm, flow rate: 0.5 ml/min.]

Retention time of main peak: around 1.9 minutes.

6. A method as claimed in claim 1 wherein the reaction product has the following physicochemical properties:

(1) IR absorption spectra by KBr tablet method:

Absorption band at around 3420, 1630, 1240, 1110 and 620 cm$^{-1}$.

(2) High performance liquid chromatogram:

[conditions; stationary phase:

octadecanylated silica-gel, 15 cm x 4.6 mm$\phi$, mobile phase: methanol : water = 40 : 60, column temperature: 25°C, detector: UV 254 nm, flow rate: 0.5 ml/min.]

Retention time of main peak: around 1.8 minutes.

7. A method as claimed in claim 1 wherein purified reaction product in a therapeutically effective amount or a pharmaceutically acceptable salt thereof is mixed with a pharmaceutically acceptable carrier therefor to provide a pharmaceutical composition.

8. An anti-retrovirus agent comprising a reaction product of Amphortericin B with a sulfonating agent, or a pharmaceutically acceptable salt thereof as an active therapeutic substance.

9. The use of the agent of claim 8 for the manufacture of a medicament for the therapeutic treatment of the acquired immune deficiency syndrome (AIDS).

10. The use of the agent of claim 8 for the manufacture of a medicament for the therapeutic treatment of the human immunodeficiency virus (HIV) infection.

Fig. 2

Retention Time (min.)

Fig. 3

Transmittance (%) vs Wavenumber (cm$^{-1}$)

EP 0 351 173 A2

Fig. 4

Retention Time (min.)